(19) Europäisches Patentamt

European Patent Office

Office européen des brevets

(11) Publication number: **0 448 592 B1**

# EUROPEAN PATENT SPECIFICATION

(45) Date of publication of patent specification: **09.03.94**   (51) Int. Cl.5: **A61M 1/14, A61M 5/142**

(21) Application number: **90900286.7**

(22) Date of filing: **13.12.89**

(86) International application number:
**PCT/GB89/01486**

(87) International publication number:
**WO 90/06781 (28.06.90 90/15)**

(54) **FLUID FLOW CONTROL APPARATUS.**

(30) Priority: **13.12.88 GB 8829311**

(43) Date of publication of application:
**02.10.91 Bulletin 91/40**

(45) Publication of the grant of the patent:
**09.03.94 Bulletin 94/10**

(84) Designated Contracting States:
**AT BE CH DE ES FR GB IT LI LU NL SE**

(56) References cited:
**EP-A- 0 120 284**
**GB-A- 2 012 373**
**US-A- 3 774 762**
**US-A- 4 226 751**

(73) Proprietor: **BIO-FLO LIMITED**
**32 St Andrews Road**
**Glasgow G41 1ST(GB)**

(72) Inventor: **HOOD, Robert Gordon**
**"Koinonia",**
**10 Balgonie Woods**
**Paisley, PA2 6HW(GB)**

(74) Representative: **Naismith, Robert Stewart et al**
**CRUIKSHANK & FAIRWEATHER**
**19 Royal Exchange Square**
**Glasgow, G1 3AE Scotland (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to fluid flow control apparatus and particularly, but not exclusively, to fluid control apparatus for use in separation and filtration systems.

Fluid flow control apparatus for use in separation and filtration systems and similar fluid handling systems such as a life support system should satisfy a number of desirable criteria in addition to being efficient and relatively inexpensive. The constituent parts of the fluid control apparatus should be easily cleaned or sterilised or be disposable as this is important when using biological fluids such as blood. The apparatus should require minimal fluid volume for analyses and should accommodate sensors for monitoring various fluid parameters of the fluid. The apparatus should also permit control of fluid flow rate and pressure to suit specific separation and filtration requirements.

Existing separation and filtration equipment does not fulfil one or more of the above mentioned requirements. In particular, existing equipment does not facilitate sterilisation requiring disassembling of components and such equipment and often requiring substantial volumes of fluid for monitoring purposes. These drawbacks are particularly evident when dealing with the treatment of biological fluids, for example, blood in dialysis or life support systems. In addition, control of flow rate and pressure is often complex in existing equipment and further reduces the sterilisability of the equipment. Thus, most existing systems tend to be aseptic rather than sterile, and this is less desirable in a clinical environment.

US-A-3 774 762 discloses a device for performing fluid processing procedures adapted to hemodialysis. The device includes a disposable component formed by layers of transparent, flexible plastic sheets, sealed to each other according to a predetermined pattern defining between each pair flow paths and pockets which respectively form fluid passages and reservoirs. The device also includes a permanent installation which is to receive the disposable component in order to form therewith a complete apparatus for automatically processing fluids. Retractable plungers are provided on the permanent installation and may be advanced to close selected flow paths for determining the pathway and timing of the fluid flows.

According to a first aspect of the present invention there is provided fluid flow control apparatus for use with a fluid handling system, said apparatus comprising a fixed portion, and a sterilisable or disposable portion removably engageable with said fixed portion, said removable portion including a deformable fluid conduit, and said flow control apparatus includes flow restriction means for restricting flow in said deformable conduit, said removable portion being adapted to be coupled to pump means and to conduits in said fluid handling system and said fluid flow control apparatus having fluid pathways for permitting fluid to flow between inlet and output ports of said fluid flow control apparatus, characterised in that said flow restriction means comprises an element on the fixed portion which co-operates with an element on the removable portion to compress said deformable conduit to define a nip when said removable portion is engaged with said fixed portion, to set the pressure in said fluid handling system.

The said deformable conduit may be a separate flexible conduit adapted to be coupled between two ports on said removable portion or an integral moulded conduit disposed between two ports in said removable portion.

The fixed portion may define a recess for slidably receiving said removable portion. However, the removable portion can be engaged with said fixed portion by a clamp or other suitable fastener.

The fixed portion may consist of two separate parts, a first guide part and a second flow restriction part, the first guide part defining the recess for slidably receiving the removable portion and the second flow restriction part being provided with an element engaging with a reaction element on said removable portion so that the deformable conduit is disposed therebetween when the fixed and removable parts are engaged.

The fluid flow control apparatus conveniently includes a fluid monitoring system, said fluid monitoring system comprising a conduit disposed in said removable portion which communicates with a main fluid conduit and said conduit having an outlet port, said first guide part having a first inlet port which sealably registers with said outlet port with which said removable portion is engaged, said first inlet port being the inlet of a sampling conduit, said sampling conduit communicating with at least one aperture for receiving a sensor for measuring a fluid parameter, said sampled fluid being passed to an outlet in said first guide part. A plurality of sensors may be removably coupled to said first guide port for sensing various fluid parameters.

The fluid flow control apparatus may be used with a separation or filtration system such as a dialysis system for detoxifying blood or in a life support system.

The removable portion and filter system can be combined to form a single integral cassette which is disposable or sterilisable.

The fluid flow control apparatus may be used in a dialysis system, with the apparatus adapted to receive blood to be treated, said control apparatus having a sterilisable removable portion being coupled to pump means and to a separation or filtration element, said fluid flow restriction means being arranged to set a level of back pressure required for dialysis, blood monitoring means coupled to said sterilisable removable portion for receiving a sample of blood being passed to said separation or filtration element for analysing at least one parameter of said blood, and purge means coupled to said flow control apparatus for being coupled to a supply of purge fluid after said dialysis to purge said blood monitoring means of blood, and control means coupled to said control unit and to said pump means and said purge means for controlling the dialysis operation and setting the blood flow rate and separation pressure.

The fluid flow control apparatus can include pressure control means for controlling the transmembrane pressure in a filtration device, the pressure control means comprising filter means having an inlet for receiving an inlet fluid to be filtered and an outlet for receiving the concentrate from said filter means, first pressure monitoring means associated with an inlet conduit for measuring the inlet pressure to said filter means, second pressure monitoring means associated with the outlet conduit for measuring the pressure of the outlet fluid, means for comparing the inlet and outlet pressures measured to provide a comparison signal, and flow control means responsive to the comparison signal to control the flow of fluid through said filter unit to optimise control of the transmembrane pressure and filtration.

The fluid flow control means may include a pressure switch located in the inlet conduit or it can be located in the outlet conduit.

The pressure monitoring means, in the form of sensors, can be disposed in a bleed line of the inlet and outlet conduits so that the fluid flows past the sensors, or the pressure sensors can be diaphragm pressure sensors so that there is no contact between the fluid being treated and the pressure monitoring apparatus. The transmembrane pressure arrangement can be used in a plasma separation system having a first pump at said filter inlet, a second pump disposed at the filter outlet and a third pump disposed at the filtrate outlet, the filtrate outlet being adapted to be coupled to a reservoir of an anticoagulent substance and said filtrate output being connected to a plasma collection unit, the apparatus being arranged to separate plasma from blood donated by a patient, whereby in use, a first value of transmembrane pressure is used to actuate the first and third pumps to separate plasma and to store the separated plasma collection unit and a second value of transmembrane pressure is used to stop the first and third pumps and to actuate the second pump to return the blood to the patient.

These and other aspects of the present invention will become apparent from the following description when taken in combination with the accompanying drawings in which:-

Fig. 1 is a perspective view of a fluid separation system incorporating fluid control apparatus in accordance with an embodiment of the the present invention;

Fig. 2 is an elevational view of part of the apparatus shown in Fig.1 taken in the direction of arrow 2 and drawn to a larger scale;

Fig. 3 is a perspective and partly exploded view of parts of the apparatus shown in Fig. 1;

Fig. 4 is an elevational view of the apparatus of Fig. 3 taken in the direction of arrow 4 in Fig. 3;

Fig. 5 is a plan view of the apparatus of Fig. 3 taken in the direction of arrow 5;

Fig. 6 is a partly assembled and perspective view of the fluid control apparatus shown in Figs. 2 to 5, and

Fig 7. and 7a depicts an embodiment of a fluid flow control apparatus in which the filter and cassette are combined in a single cassette;

Fig. 8 is a diagrammatic representation of an embodiment of fluid flow control apparatus according to the present invention used in a transmembrane pressure monitoring application, and

Fig. 9 is a diagrammatic representation of a further embodiment of fluid flow control apparatus according to the present invention using transmembrane pressure monitoring to control donor plasma separation.

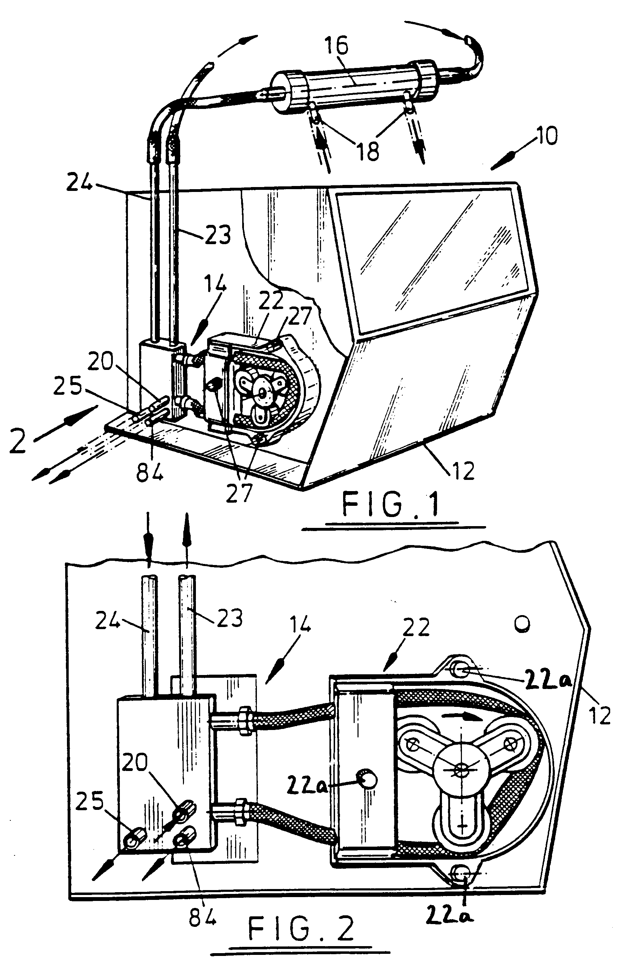

Reference is first made to Figs. 1 and 2 of the drawings which shows a fluid separation system generally indicated by reference numeral 10. The fluid separation system 10 is depicted in use in a dialysis system where blood is taken from a patient and filtered through a semi-permeable membrane to remove waste products in the blood before the treated blood is returned to the patient. The system consists of a fluid control unit 12 incoporating fluid flow control apparatus 14 which receives blood from a patient and which passes the blood through a hollow fibre dialysis membrane 16 which is also connected via ports 18 to a dialysate reservoir (not shown) and the filtered or treated blood is returned to the patient via the fluid flow control apparatus as will be later described in detail.

Blood enters the apparatus 14 through main feed port 20 and is subsequently pumped through the apparatus by a 3-lobe peristaltic pump 22, along conduit 23 to the hollow fibre membrane 16. Filtered blood is returned via conduit 24 through fluid control apparatus 14 and is then returned to the patient via outlet tube 25. Parts of the fluid control unit 12, the tube in the peristaltic pump and filtration unit 16 are removable for sterilisation as will be described. The fluid control apparatus also comprises a sterilising purge system, a fluid monitoring arrangement and a transmembrane pressure monitoring system as will also be described.

The flow rate and pressure of blood in the system is controlled by pump 22 and flow control apparatus 14. The operation of pump 22 is well known and forms no part of the invention. The pump cover can be removed using fasteners 22a to allow various parts to be replaced or sterilised if required.

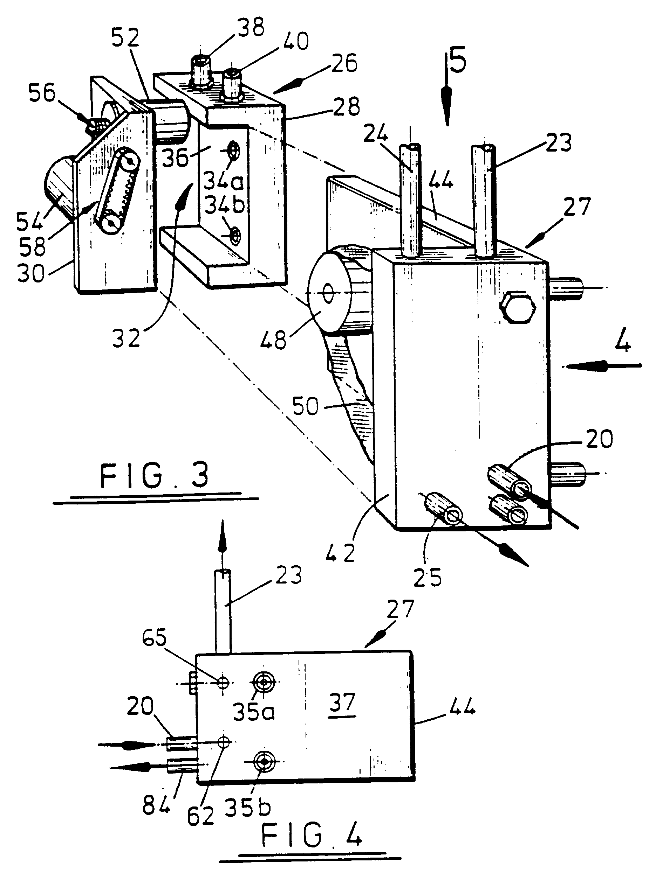

Reference is now made to Figs. 3, 4 and 5 of the drawings which depict the fluid flow control apparatus 14 in greater detail. The fluid flow control apparatus 14 consists of two parts: a first main body portion, generally indicated by reference numeral 26, which remains fixed in the BIO 2000 (Trade Mark) control unit 12 and a removable cassette element 27. The main body portion 26 has two parts; a cassette guide 28 and a separate valve control part 30. The guide 28 and valve control part 30 are spaced apart to define a channel 32 for slidably receiving the removable cassette element 27 in a close fitting arrangement.

The guide 28 has aseptic ports 34a, b disposed on its inner surface 36 for registering with like ports 35a, b as disposed on the outer surface 37 of cassette element 27, as best seen in Fig. 4. In the assembled condition a pressure sensor 38 and pressure switch 40 are securely fastened in the top of the guide 28 to monitor this fluid pressure.

The cassette element 27 is generally L-shaped in plan and consists of machined blocks 42 and 44 of medical grade sterilisable stainless steel which are of sufficient thickness to accommodate fluid flow channels as will be fully explained later. The cassette element 27 contains the apparatus inlet port 20 and various other inlets and outlets as will also be explained. The cassette also includes an element which is part of the control valve 30. This is a fixed cylindrical boss 48 around which is located a flexible conduit 50 which connects the conduit 24 with the unit outlet tube 25. The main part of control value 30 includes a rotable cam 52 coupled to an electric motor 54 via gears 56 and a chain and sprocket drive 58. When the cassette element 27 is in an assembled position as in Fig. 1 or 2, the electric motor is operable by a central control unit, (not shown) within apparatus 10, to control the position of cam 52 so that the distance between the cam 52 and boss 48 is controlled to define a 'nip' on tube 50. This nip is used to create the back pressure in the separation element or filter 16 to suit the filtration requirements of the blood.

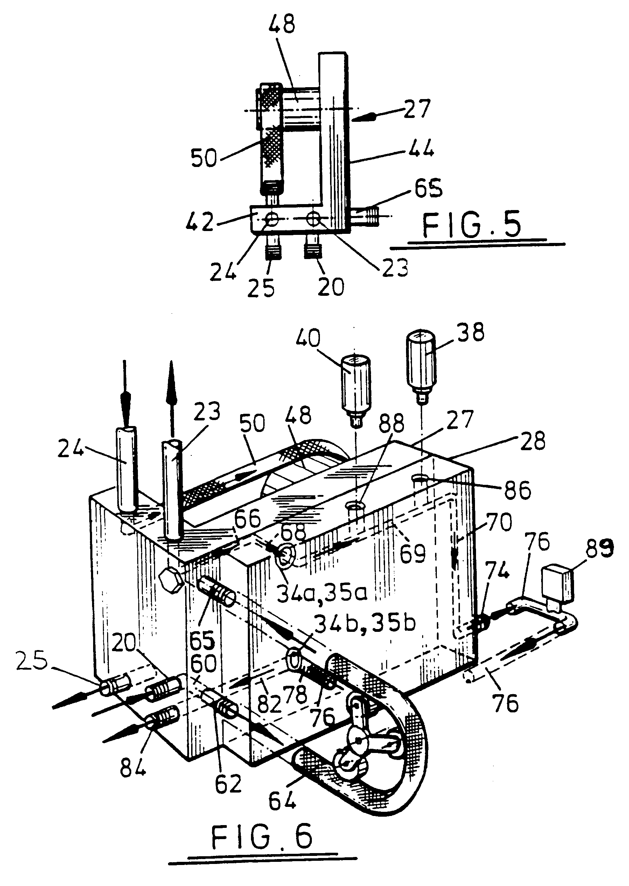

Reference is now made to Fig. 6 of the drawings and for ease of understanding the blood flow path will be followed first. Blood to be treated is passed into the cassette element 27 via inlet 20 through internal conduit 60 (shown in broken outline) and via outlet 62 to conduit 64 of the 3-lobe peristaltic pump 22. The pumped blood is passed back to port 65 and, via internal conduit 66 the main volume of blood is passed through conduit 23 to the hollow fibre dialyser 16 for filtration as described below. A smaller volume of blood is passed along internal conduit 68 and aseptic ports 34a, 35a to internal conduit 69 disposed in the cassette guide 28. The internal conduit 69 travels downwardly in portion 70 and then exits guide 28 element via nozzle 74. To nozzle 74 is connected a conduit 76, the other end of which connects to a nozzle 78 disposed in the outer face of guide 28 where it passes through aseptic ports 35b, 34b respectively to internal conduit 82 which leads to the outlet port 84.

There are drilled apertures, 86, 88 in the top of the cassette guide 28 which meet with internal conduit 69. The apertures receive sensors 38, 40 so that the leading ends of the sensors are disposed within the conduit 69 for monitoring blood parameters, in this case pH and temperature. A solenoid valve 89 is disposed in conduit 76 for controlling the start and finish of a purge operation as will be explained.

In operation, the system is connected up as shown in Fig. 1 and the system primed with blood in the usual manner. The control valve 30 is opened as is solenoid valve 89 so that a volume of blood passes through the cassette 27. Pump 22 is then started to create flow and fluid is checked in the entire system. The pump speed is then set to provide the flow conditions appropriate for separation. The control valve 30 is then adjusted to set the back pressure appropriate to the membrane for filtration requirements. During operation pressure sensor 38, monitors the fluid pressure and control pressure switch 40.

In order to stop operation, the patient is disconnected and the main valve 30 and solenoid valve 89 are opened. A purging fluid is connected to inlet port 20 and outlet port 84 and the residue of blood is flushed out of the dialysate system and the fluid flow control system. The pump is then stopped but the system can continue to be purged or to drain. The cassette element 27 is readily removably by sliding into and out of engagement with the fixed guide 28 and the conduit 50 can be removed to allow the cassette element 27 to be sterilised. The aseptic ports 34a,b, 35a, b, ensure that the biological fluid is not contaminated and complies with existing safety requirements.

Various modifications can be made to the embodiment hereinbefore described without departing from the scope of the present invention. Any fluid having components which require to be filtered or separated can be used. Any other suitable form of membrane separator can be used, such as spirally wound or flat membranes as well as hollow fibre membranes. Other fluid parameters can be monitored using appropriate sensors instead of the pressure sensor and switch, for example viscosity and conductivity. The cassette can be made of sterilisable plastic instead of stainless steel and can also be disposable.

The conduit 50 need not be external but may be integral with a moulded plastic cassette but still being deformable in response to the cam to effect flow restriction. Although the fluid pathways in the removable cassette are internal it will be understood that the pathways could be external and in the form of tubes for example.

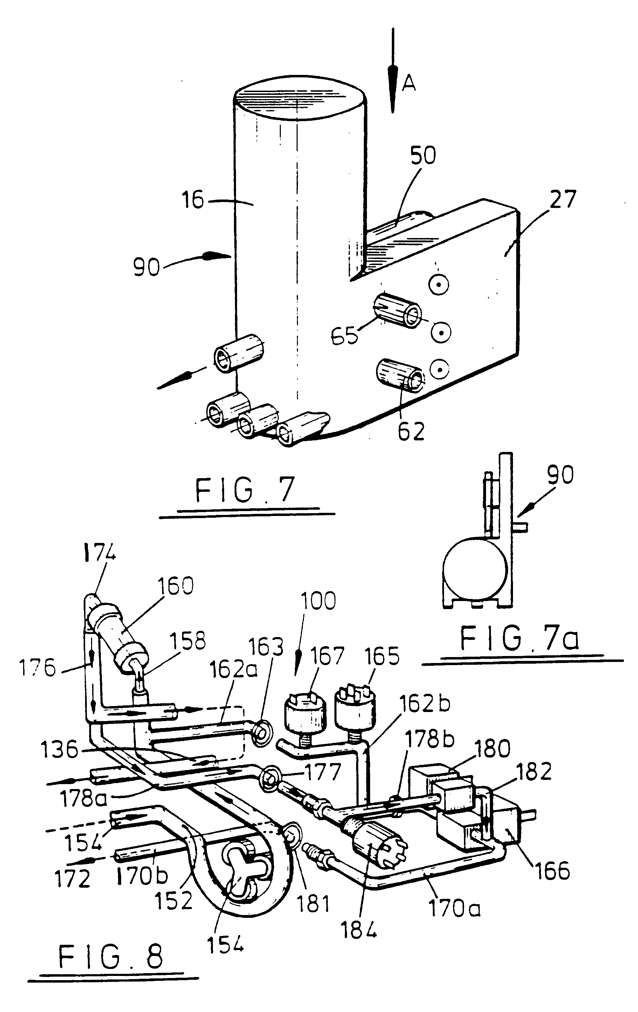

The cassette can be made as a unitary item; the filter 16 and lines can be combined with cassette 27 in an integral single unit 90 as best seen in the embodiment shown in Figs. 7 and 7a. In this case the entire cassette assembly may be disposable or sterilisable.

In addition the flow restriction means could be disposed at any suitable location rather than in the flow control unit. For example the flow restriction means could be near or at the dialyser or the peristaltic pump. Control of the flow restriction means may be by a manual pinch valve instead of the motor controlled operation. The cassette could be engaged with the fixed part other than by sliding, for example it could be placed in proximity to the fixed part and held thereat by a clamp. Furthermore the monitoring and purge system are not essential to every embodiment but only where an analysis of the fluid is required such as in the aforedescribed embodiment. Nevertheless, it is desirable to monitor certain parameters of most fluids being treated.

Reference is now made to Figure 8 of the drawings which depicts a fluid circuit generally indicated by reference number 100 for use with a BIO 2000 filtration system (Bio-flow Ltd., U.K.) which can also be used with the embodiments of Figs. 7, 7a. In the system shown in Figure 8 a fluid conduit 152 has an inlet for receiving fluid to be treated. A three lobe roller pump 154 forces the fluid along conduit 152 and through an inlet 158 to a hollow fibre filter unit generally shown by reference numeral 160. A bleed line 162a is taken from the inlet conduit 152 so that the inlet fluid can be monitored by various sensors. In the example shown, the pressure transducer 165 and pressure switch 167 are located in the bleed line 162b for measuring the pressure parameters of the inlet fluid. The bleed line is fed to a solenoid valve 166, the output of which is connected to a conduit 170a which, in turn, is coupled via outlet 172 to the system being treated.

The filter unit 160 has a filter output 174 which feeds outlet conduit 176 which passes through a pressure control valve, not shown in the interest of clarity, and the output from the valve constitutes the return fluid which is fed back to the source, in this example the vascular system of a patient. The outlet conduit has a bleed line 178 which is connected to a second solenoid valve 180 which has an output 182 which feeds into conduit 170 as described above. A pressure transducer 184 is located in the bleed line 178 for measuring the pressure of the filter output fluid. It will be understood that conduits 162a, 178a and 170b are located in the removable portion 14 or cassette of the BI0 2000 filtration system when these conduits are coupled via aseptic ports 163, 177 and 181 to conduits 162b, 178b and 170a respectively which are located in the fixed portion 12.

In the arrangement shown in Figure 8, the pressure switch 167 and pressure sensors 165 and 184 are located in line so that the fluid flows through and is in contact with the sensors.

With this arrangement, transmembrane pressure measurement (TMP) is achieved by monitoring the inlet pressure with transducer 165 and the outlet pressure with transducer 184. The transmembrane pressure is calculated as the average of the inlet and outlet pressures (Po + Pi/2) less the filtrate pressure ($P_f$). However, filtrate or permeate pressure is low and is generally taken as zero so that the average transmembrane pressure (TMP) is deemed to be the average of the inlet and outlet pressures. The measured pressures are processed in a routine manner to provide the average pressure used to control pressure switch 167 to vary the flow through the solenoid or to affect the back pressure of the system or the signal can be used to control the valve to vary the pressure in the filter outlet conduit by varying the distance between the cam and boss and thus affect the transmembrane pressure. With this arrangement, control of the transmembrane pressure is optimised to minimise effects of gel polarisation and the like which is a process which is known to slow down filtration.

A system generally similar to that shown in Figure 8 can be used except that pressure is measured using diaphragm pressures sensors as opposed to a flow-through system which means that the fluid being filtered does not contact the monitoring sensor which maintains the sterility and the integrity of the fluid being treated.

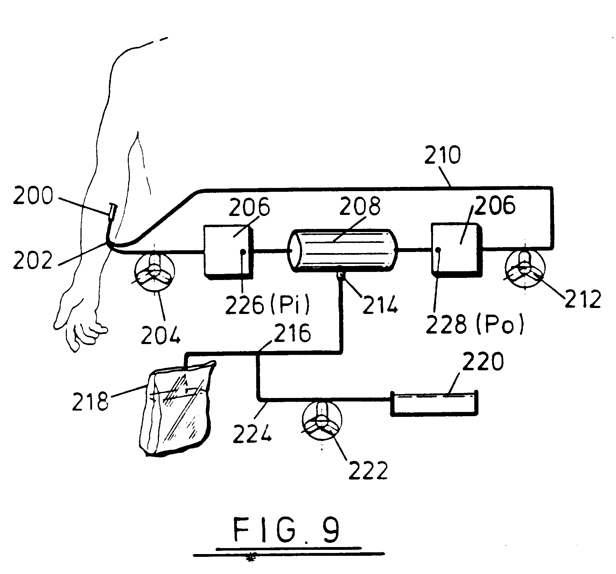

An embodiment of the present invention set up for monitoring transmembrane pressure (TMP) will now be described in an application for controlling plasma donor separation and this is diagrammatically shown in Fig. 9

In this arrangement a vein of a patient is cannulated with a single needle shunt 200 and the blood is pumped through 2-way line 202 by a fast 3-lobe pump 204 through fluid flow control apparatus, general indicated by reference numeral 206 through hollow fibre filter 208 and back through the apparatus 206. The filter outlet line 210 is passed through a second 3-lobe pump 212 and is coupled via 2-way line 202 to the patient. The filtrate outlet 214 of the filter is coupled via fluid line 216 to a plasma collection bag 218 and anticoagulent is pumped from a reservoir 220 by a third 3-lobe pump 222 via line 224 into line 216 to prevent the plasma from coagulating.

The fluid control apparatus 206 has inlet and output pressure sensors 226, 228 which are used to monitor the transmembrane pressure (TMP) for controlling the plasma donor separation as will be described.

In operation the system is set up as shown and pump 204 is actuated to pump blood from the patient through the system. As the blood passes through the filter it is pumped by pump 222 to bag 218 while being combined with the anticoagulent. As the blood passes to the filter retentate outlet the inlet pressure ($P_i$) and outlet pressure ($P_o$) are monitored by sensors 226, 228 respectively and the transmembrane pressure (TMP) monitored.

When the TMP reaches a first preset value pumps 204, 222 are switched off and pump 212 switched on to return the cells in the fluid retentate back to the patient via lines 210 and 202. The TMP continues to be monitored and as the pressure falls, a second preset value is reached so that pumps 204, 222 are switched on and pump 212 is switched off to continue with further plasma separation. These procedures are repeated until sufficient plasma is separated.

It will be understood that the pressures in this application are monitored using the aforementioned pressure diaphragm sensors to avoid contact between the blood and the pressure monitoring circuitry which is an advantage of this arrangement. A further advantage is that no valves are required to control flow, this being achieved solely using the three pumps, which act as valves. Another advantage is that the total fluid line including the filter and bag is disposable. In this arrangement air bubble detectors are provided in the feed and return lines and, in the event of a bubble being detected, the whole system is shut down.

The apparatus hereinbefore described has application in other clinical areas such as life support systems as well as in the separation of substances from non-biological fluids, for example rare earth element or mineral separation. It will also be understood that the apparatus hereinbefore described can be used in different sizes, i.e. it can be scaled up to industrial plant size.

Advantages associated with the present invention are that the cassette is readily removeable to facilitate sterilisation and the fluid volume required for monitoring is very small, being less than 40ml. The cassette can be disposable as can the entire filter and assembly in one embodiment. Also multipoint sensor access is provided to monitor various fluid parameters, and the sensors can be easily removed or replaced. In addition the pressure control for setting the filtration conditions is non-invasive and no contact is made with the fluid being treated which is important when using bioligical fluids. The transmembrane filtration/separation pressure can be readily and easily varied to suit specific filtration/separation applications and the equipment can be used with biological and non-biological fluids in various applications.

## Claims

1. Fluid flow control apparatus for use with a fluid handling system (10), said apparatus (14) comprising a fixed portion (26), and a sterilisable or disposable portion (27) removably engageable with said fixed portion, said removable portion (27) including a deformable fluid conduit (50), and said flow control apparatus includes flow restriction means (48, 52) for restricting flow in said deformable conduit (50), said removable portion (27) being adapted to be coupled to pump means (22) and to conduits (23, 24) in said fluid handling system and said fluid flow control apparatus having fluid pathways (60, 66, 68, 69, 82, 84) for permitting fluid to flow between inlet and output ports (20, 25) of said fluid flow control apparatus, characterised in that said flow restriction means comprises an element (52) on the fixed portion (26) which cooperates with an element (48) on the removable portion (27) to compress said deformable conduit to define a nip when said removable portion is engaged with said fixed portion (26), to set the pressure in said fluid handling system (10).

2. Fluid control apparatus as claimed in claim 1 wherein said deformable conduit (50) is a separate flexible conduit adapted to be coupled between two ports on said removable portion (27).

3. Fluid control apparatus as claimed in claim 1 wherein said deformable conduit is an integral moulded conduit disposed between two ports in said removable portion.

4. Fluid control apparatus as claimed in any preceding claim wherein the fixed portion (26) defines a recess (32) for slidably receiving said removable portion (27).

5. Fluid control apparatus as claimed in any one of claims 1 to 4 wherein the removable portion can be engaged with said fixed portion by a clamp or other suitable fastener.

6. Fluid control apparatus as claimed in any preceding claim wherein the fixed portion (26) consists of two separate parts, a first guide part (28) and a second flow restriction part (30), the first guide part (28) defining a recess (32) for slidably receiving the removable portion (27) and the second flow restriction part (30) being provided with an element (52) engaging with a reaction element (48) on said removable portion (27) so that the deformable conduit (50) is disposed therebetween when the fixed and removable portions are engaged.

7. Fluid control apparatus as claimed in claim 6 wherein said fluid flow control apparatus includes a fluid monitoring system, said fluid monitoring system comprising a conduit (68) disposed in said removable portion (27) which communicates with a main fluid conduit (66), said conduit (68) having an outlet port (35a), said first guide part (28) having a first inlet port (34a) which sealably registers with said outlet port (35a) with which said removable portion (27) is engaged, said first inlet port (34a) being the inlet of a sampling conduit (69), said sampling conduit (69) communicating with at least one aperture (86, 88) for receiving a sensor (38, 40) for measuring a fluid parameter, said sampled fluid being passed to an outlet (74) in said first guide part (28).

8. Fluid control apparatus as claimed in claim 7 wherein a plurality of sensors (38, 40) are removably coupled to said first guide part (28) for sensing various fluid parameters.

9. Fluid control apparatus as claimed in claim 7 or claim 8 wherein said outlet (74) in said first guide part (28) is coupled via valve means (89) and a conduit (76) to a second inlet (78) in said guide part (28), said second inlet (78) in said guide part being connected via internal conduits and third and fourth ports (34b, 35b) back to said removable portion (27) and to an outlet purge port (84), the arrangement being such that purging fluid can be passed through said removable portion (27) and said first guide part (28) when said valve (89) is opened.

10. Fluid control apparatus as claimed in any preceding claim wherein said fluid flow control apparatus is used with a separation or filtration system.

11. Fluid control apparatus as claimed in claim 10 wherein the separation system is a dialysis system for detoxifying blood.

12. Fluid control apparatus as claimed in claim 10 wherein said fluid flow control apparatus is used with a life support system.

13. Fluid control apparatus as claimed in any one of claims 9 to 12 wherein said outlet port (35a), said first inlet port (34a) and said third and fourth inlet and outlet ports (34b, 35b) are aseptic ports.

14. Fluid control apparatus as claimed in any one of claims 7 to 13 wherein said removable part (27) is made of medical grade stainless steel.

15. Fluid control apparatus as claimed in any one of claims 7 to 13 wherein said removable part (27) is made of sterilisable plastic.

16. Fluid control apparatus as claimed in any preceding claim wherein the removable part (27) and a filter unit (16) are combined in a single integral structure (90) which is removably coupled to the fixed part.

17. A fluid separation or filtration system for receiving a fluid having at least one substance to be separated therefrom, said system comprising a fluid flow control apparatus as claimed in any preceding claim, pump means (22) for pumping said fluid around said system, and separation or filtration means (16) for separating said substance from said fluid.

18. A system as claimed in claim 17 wherein said flow restriction means (48, 52) associated with said flow control apparatus (14) is adapted for setting the back pressure in said separation means.

19. A system as claimed in claim 17 or 18 including fluid monitoring means (38, 40) coupled to said flow control apparatus (14) for monitoring at least one fluid parameter.

20. A system as claimed in any one of claims 17 to 19 including a purge system for purging fluid from said fluid monitoring means (38, 40), said purge system being coupled to said fluid flow control apparatus (14) and including valve means (89).

21. A system as claimed in any one of claims 17 to 20 wherein the separation system is a dialysis system and the flow control apparatus removable sterlisable portion (27) is slidably coupled to a guide means (28) of said fixed portion (26) via aseptic ports (34a, 35a, 34b, 35b) whereby a sample of blood can be passed to said monitoring means (38, 40) for analysis.

22. Fluid flow control apparatus as claimed in claim 1 for use in a dialysis system for receiving blood to be treated, said sterlisable removable portion (27) being coupled to pump means (22) and to a separation or filtration element (16), said flow restriction means (48, 52) being arranged to set a level of back pressure required for dialysis, blood monitoring means (38, 40) coupled to said sterilisable removable portion (27) for receiving a sample of blood being passed to said separation or filtration (16) element for analysing at least one parameter of said blood, and purge means coupled to said flow control apparatus for being coupled to a supply of purge fluid after said dialysis to purge said blood monitoring means (38, 40) of blood, and control means coupled to said control apparatus, to said pump means (22) and to said purge means for controlling the dialysis operation and setting the blood flow rate and separation pressure.

23. Fluid flow control apparatus as claimed in claim 1 including pressure control means for controlling the transmembrane pressure in a filtration device, the pressure control means comprising filter means (160) having an inlet (158) for receiving an inlet fluid to be filtered and an outlet (174) for receiving the concentrate from said filter means (160), first pressure monitoring means (165) associated with an inlet conduit for measuring the inlet pressure to said filter means, second pressure monitoring means (184) associated with an outlet conduit for measuring the pressure of the outlet fluid, means for comparing the inlet and outlet pressures measured to provide a comparison signal, and flow control means (48, 52) responsive to the comparison signal to control the flow of fluid through said filter unit (160) to optimise control of the transmembrane pressure and filtration.

24. Fluid flow control apparatus as claimed in claim 23 wherein the fluid flow control means includes a pressure switch (167) located in the inlet conduit.

25. Fluid flow control apparatus as claimed in claim 23 wherein fluid flow control means includes a pressure switch is located in the outlet conduit.

26. Fluid control apparatus as claimed in any one of claims 23 to 25 wherein the pressure monitoring means (165, 184) are disposed in respective bleed lines (162b, 178b) of the inlet and outlet conduits so that the fluid flows past the monitoring means.

27. Fluid flow control apparatus as claimed in any one of claims 23 to 25 wherein the pressure monitoring means (165, 184) are diaphragm pressure sensors so that there is no contact between the fluid being treated and the pressure monitoring apparatus.

28. Fluid flow control apparatus as claimed in any one of claims 23 to 27 wherein the first and second pressure monitoring means (165, 184) are coupled to first and second solenoid valves (166, 180) respectively, the outputs of which are combined within a single conduit (170a), the valves (166, 180) being actuatable to purge fluid from an associated measurement fluid circuit.

29. Fluid flow control apparatus as claimed in any one of claims 23 to 28 having a first pump at said filter inlet, a second pump disposed at the filter outlet and a third pump disposed at the filtrate outlet, the filtrate outlet being adapted to be coupled to a reservoir of an anticoagulent substance and said filtrate output being connected to a plasma collection unit, the apparatus being arranged to separate plasma from blood donated by a patient, whereby in use, a first value of transmembrane pressure is used to actuate the first and third pumps to separate plasma and to store the separated plasma in a collection unit and a second value of transmembrane pressure is used to stop the first and third pumps and to actuate the second pump to return the blood to the patient.

**Patentansprüche**

1. Fluidströmungs-Steuergerät, das für die Verwendung mit einem Fluidhandhabungssystem (10) bestimmt ist, wobei dieses Steuergerät (14) eine fest eingebaute Komponente (26) und eine mit der fest eingebauten Komponente herausnehmbar verbindbare, sterilisierbare oder wegwerfbare Komponente (27) aufweist, wobei die herausnehmbare Komponente (27) eine verformbare Fluidleitung (50) umfaßt, und dieses Steuergerät (14) ein Strömungsbegrenzungsmittel (48, 52) umfaßt, um die Strömung in der verformbaren Leitung (50) zu begrenzen, die herausnehmbare Komponente (27) ausgelegt ist, um mit einem Pumpenmittel (22) und Leitungen (23, 24) in dem Fluidhandhabungssystem verbunden zu werden, und dieses Fluidströmungs-Steuergerät (14) Fluidkanäle (60, 66, 68, 69, 82, 84) aufweist, um dem Fluid zu ermöglichen, zwischen einer Einlaßöffnung und einer Auslaßöffnung (20, 25) des Fluidströmungs-Steuergerätes (14) zu strömen, dadurch gekennzeichnet, daß das Strömungsbegrenzungsmittel ein Element (52) auf der fest eingebauten Komponente (26) aufweist, das dann, wenn die herausnehmbare Komponente in die fest eingebaute Komponente (26) eingeschoben ist, mit einem Element (48) auf der herausnehmbaren Komponente (27) zusammenwirkt, um die verformbare Leitung zusammenzudrücken und eine Klemmstelle festzulegen, über die der Druck in dem Fluidhandhabungssystem (10) eingestellt wird.

2. Fluidströmungs-Steuergerät gemaß Anspruch 1, wobei die verformbare Leitung (50) eine getrennte, flexible Leitung ist, die ausgelegt ist, um mit zwei Öffnungen auf der herausnehmbaren Komponente (27) verbunden zu werden.

3. Fluidströmungs-Steuergerät gemäß Anspruch 1, wobei die verformbare Leitung eine integral geformte Leitung ist, die zwischen zwei Öffnungen in der herausnehmbaren Komponente angeordnet ist.

4. Fluidströmungs-Steuergerät gemäß irgendeinem vorhergehenden Anspruch, wobei die fest eingebaute Komponente (26) eine Aussparung (32) festlegt, um die herausnehmbare Komponente (27) verschiebbar aufzunehmen.

5. Fluidströmungs-Steuergerät gemäß, irgendeinem der Ansprüche 1 bis 4, wobei die herausnehmbare Komponente mittels einer Klammer oder eines anderen, geeigneten Befestigungselements mit der fest eingebauten Komponente fest verbunden werden kann.

6. Fluidströmungs-Steuergerät gemäß irgendeinem vorhergehenden Anspruch, wobei die fest eingebaute Komponente (26) aus zwei getrennten Teilen besteht, und zwar einem ersten Führungsteil (28), und einem zweiten Strömungsbegrenzungsteil (30), wobei das erste Führungsteil (28) eine Aussparung (32) festlegt, um die herausnehmbare Komponente (27) verschiebbar aufzunehmen, und das zweite Strömungsbegrenzungsteil (30) mit einem Element (52) versehen ist, das dann, wenn die fest eingebaute Komponente in die herausnehmbare Komponente eingeschoben ist, mit einem Rückwirkungselement (48) auf der herausnehmbaren Komponente (27) so zusammenwirkt, daß die verformbare Leitung (50) dazwischen angeordnet ist.

7. Fluidströmungs-Steuergerat gemäß Anspruch 6, das ein Fluidüberwachungssystem umfaßt, wobei dieses Fluidüberwachungssystem eine in der herausnehmbaren Komponente (27) angeordnete Leitung (68) aufweist, die mit einer Fluid-Hauptleitung (66) in Verbindung steht, die Leitung (68) eine Auslaßöffnung (35a) hat, das erste Führungsteil (28) eine erste Einlaßöffnung (34a) hat, die bei dichter Verbindung genau auf die mit der herausnehmbaren Komponente (27) verbundene Auslaßöffnung (35a) paßt, die erste Einlaßöffnung (34a) der Einlaß einer Probenentnahme-Leitung (69) ist, die Probenentnahme-Leitung (69) mit mindestens einer Öffnung (86, 88) in Verbindung steht, um einen Fühler (38, 40) zum Messen eines Fluidparameters aufzunehmen, und das entnommene Fluid zu einem Auslaß (74) in dem ersten Führungsteil (28) weitergeleitet wird.

8. Fluidströmungs-Steuergerät gemäß Anspruch 7, wobei eine Vielzahl von Fühlern (38, 40) herausnehmbar mit dem ersten Führungsteil (28) verbunden ist, um verschiedene Fluidparameter zu fühlen.

9. Fluidströmungs-Steuergerät gemäß Anspruch 7 oder 8, wobei der Auslaß (74) in dem ersten Führungsteil (28) über ein Ventilmittel (89) und eine Leitung (76) mit einem zweiten Einlaß (78) in dem Führungsteil (28) verbunden ist, der zweite Einlaß (78) in dem Fuhrungsteil über innere Kanäle und eine dritte und vierte Öffnung (34b, 35b) wieder mit der herausnehmbaren Komponente (27), und einer Reinigungs-Auslaßöffnung (84) verbunden ist, und die Anordnung so ausgelegt ist, daß dann, wenn das Ventil (89) geöffnet ist, ein Reinigungsfluid durch die herausnehmbaren Komponente (27) und das erste Führungsteil (28) geleitet werden kann.

10. Fluidströmungs-Steuergerät gemäß irgendeinem vorhergehenden Anspruch, das in Verbindung mit einem Abscheidungs- oder Filtrationssystem verwendet wird.

**11.** Fluidströmungs-Steuergerät gemäß Anspruch 10, wobei das Abscheidungssystem ein Dialysesystem zur Blutentgiftung ist.

**12.** Fluidströmungs-Steuergerät gemäß Anspruch 10, das in Verbindung mit einem Lebenserhaltungssystem verwendet wird.

**13.** Fluidströmungs-Steuergerät gemäß irgendeinem der Ansprüche 9 bis 12, wobei die Auslaßöffnung (35a), die erste Einlaßöffnung (34a), und die dritte und vierte Einlaß- und Auslaßöffnung (34b, 35b) aseptische Öffnungen sind.

**14.** Fluidströmungs-Steuergerät gemäß irgendeinem der Ansprüche 7 bis 13, wobei die herausnehmbare Komponente (27) aus rostfreiem Stahl von medizinischem Gütegrad besteht.

**15.** Fluidströmungs-Steuergerät gemäß irgendeinen der Ansprüche 7 bis 13, wobei die herausnehmbare Komponente (27) aus sterilisierbarem Kunststoff besteht.

**16.** Fluidströmungs-Steuergerät gemäß irgendeinem vorhergehenden Anspruch, wobei die herausnehmbare Komponente (27) und eine Filtereinheit (16) in einer einzelnen, integralen Baueinheit (90) vereinigt sind, die mit der fest eingebauten Komponente herausnehmbar verbunden ist.

**17.** Fluidabscheidungs- oder Fluidfiltrationssystem zur Aufnahme eines Fluids, das mindestens eine abzuscheidende Substanz enthält, wobei dieses System ein Fluidströmungs-Steuergerät gemäß irgendeinem vorhergehenden Anspruch, ein Pumpenmittel (22) zum Umwälzen des Fluids in dem System, und ein Abscheidungs- oder Filtrationsmittel (16) zum Abscheiden der Substanz aus dem Fluid aufweist.

**18.** System gemäß Anspruch 17, wobei das mit dem Fluidströmungs-Steuergerät (14) kombinierte Strömungsbegrenzungsmittel (48, 52) ausgelegt ist, um den Gegendruck in dem Abscheidungsmittel einzustellen.

**19.** System gemäß Anspruch 17 oder 18, das ein mit dem Fluidströmungs-Steuergerät (14) verbundenes Fluidüberwachungsmittel (38, 40) umfaßt, um mindestens einen Fluidparameter zu überwachen.

**20.** System gemäß irgendeinem der Ansprüche 17 bis 19, das ein Reinigungssystem umfaßt, um das Fluid aus dem Fluidüberwachungsmittel (38, 40) abzulassen, wobei dieses Reinigungssystem mit dem Fluidströmungs-Steuergerät (14) verbunden ist und ein Ventilmittel (89) umfaßt.

**21.** System gemäß irgendeinem der Ansprüche 17 bis 20, wobei das Abscheidungssystem ein Dialysesystem ist, und die herausnehmbare, sterilisierbare Komponente (27) des Fluidströmungs-Steuergeräts über aseptische Öffnungen (34a, 35a, 34b, 35b) mit einem Führungsteil (28) der fest eingebauten Komponente (26) verschiebbar verbundan ist, wodurch eine Probe des Bluts zwecks Analyse nach dem Überwachungsmittel (38, 40) weitergeleitet werden kann.

**22.** Fluidströmungs-Steuergerät gemäß Anspruch 1, das für die Verwendung in einem Dialysesystem zur Aufnahme von zu behandelndem Blut bestimmt ist, wobei die sterilisierbare, herausnehmbare Komponente (27) mit einem Pumpenmittel (22) und einem Abscheidungs- oder Filtrationselement (16) verbunden ist, das Strömungsbegrenzungsmittel (48, 52) ausgelegt ist, um einen für die Dialyse erforderlichen Gegendruck einzustellen, das Blutüberwachungsmittel (38, 40) mit der sterilisierbaren, herausnehmbaren Komponente (27) verbunden ist, um eine Probe des Bluts zu erhalten, das nach dem Abscheidungs- oder Filtrationselement (16) weitergeleitet wird, um mindestens einen Parameter dieses Bluts zu analysieren, und ein Reinigungsmittel mit dem Fluidströmungs-Steuergerät verbunden ist, um nach dieser Dialyse mit einem Vorrat von Reinigungsfluid verbunden zu werden, um das Blutüberwachungsmittel (38, 40) von Blut zu reinigen, und ein Steuermittel mit dem Steuergerät, dem Pumpenmittel (22), und dem Reinigungsmittel verbunden ist, um den Dialysevorgang zu steuern und die Blut-Strömungsrate und den Abscheidungsdruck einzustellen.

**23.** Fluidströmungs-Steuergerät gemäß Anspruch 1, das ein Drucksteuermittel umfaßt, um den Transmembrandruck in einer Filtrationseinrichtung zu steuern, wobei das Drucksteuermittel aufweist: ein Filtermittel (160), mit einem Einlaß (158), um ein zu filterndes Einlaßfluid aufzunehmen, und einem Auslaß (174), um das Konzentrat von dem Filtermittel (160) aufzunehmen, ein mit einer Einlaßleitung verburdenes, erstes Drucküberwachungsmittel (165), um den Einlaßdruck des Filtermittels zu messen, ein mit einer Auslaßleitung verbundenes, zweites Drucküberwachungsmittel (184), um den Druck des Auslaßfluids zu messen, ein Mittel zum Vergleichen des gemessenen Einlaßdrucks und Auslaßdrucks, um ein Vergleichssignal zu erzeugen, und ein auf das Vergleichssignal ansprechendes Strömungssteuermittel (48, 52), um die Strömung des Fluids durch die Filtereinheit (160) zu steuern, damit die Steuerung des Transmembrandrucks und der Filtration optimiert wird.

24. Fluidströmungs-Steuergerät gemäß Anspruch 23, wobei das Fluidströmungs-Steuermittel einen in der Einlaßleitung gelegenen Druckschalter (167) umfaßt.

25. Fluidströmungs-Steuergerät gemäß Anspruch 23, wobei das Fluidströmungs-Steuermittel einen in der Auslaßleitung gelegenen Druckschalter umfaßt.

26. Fluidströmungs-Steuergerät gemäß irgendeinem der Ansprüche 23 bis 25, wobei die Drucküberwachungsmittel (165, 184) in den betreffenden Entnahmeleitungen (162b, 178b) der Einlaßleitung bzw. der Auslaßleitung angeordnet sind, so daß das Fluid an den Überwachungsmitteln vorbeiströmt.

27. Fluidströmungs-Steuergerät gemäß irgendeinem der Ansprüche 23 bis 25, wobei die Drucküberwachungsmittel (165, 184) Membrandruckfühler sind, so daß kein Kontakt zwischen dem zu behandelnden Fluid und dem Drucküberwachungsgerät vorhanden ist.

28. Fluidströmungs-Steuergerät gemäß irgendeinem der Ansprüche 23 bis 27, wobei das erste und das zweite Drucküberwachungsmittel (165, 184) mit einem ersten bzw. zweiten Magnetventil (166, 180) verbunden sind, deren Ausgänge zu einer einzigen Leitung (170a) vereinigt sind, und die Ventile (166, 180) betätigbar sind, um das Fluid aus einem zugehörigen Fluid-Meßkreis abzulassen.

29. Fluidströmungs-Steuergerät gemäß irgendeinem der Ansprüche 23 bis 28, bei dem eine erste Pumpe bei dem Filtereinlaß, eine zweite Pumpe bei dem Filterauslaß, und eine dritte Pumpe bei dem Filtratauslaß angeordnet ist, wobei der Filtratauslaß ausgelegt ist, um mit einem eine gerinnungshemmende Substanz enthaltenden Vorratsbehälter verbunden zu werden, und der Filtratauslaß mit einer Plasmaauffangeinheit verbunden ist, wobei das Steuergerät ausgelegt ist, um das Plasma aus dem von einem Patienten gespendeten Blut abzuscheiden, wozu beim Gebrauch ein erster Wert des Transmembrandrucks verwendet wird, um die erste und die dritte Pumpe einzuschalten, damit das Plasma abgeschieden wird, und das abgeschiedene Plasma in einer Auffangeinheit gespeichert wird, und ein zweiter Wert des Transmembrandrucks verwendet wird, um die erste und die dritte Pumpe auszuschalten, und die zweite Pumpe einzuschalten, damit das Blut zu dem Patienten zurückströmt.

**Revendications**

1. Appareil de réglage du débit d'un fluide, destiné à être utilisé avec un système de manipulation d'un fluide (10), ledit appareil (14) comprenant une partie fixe (24) et une partie stérilisable ou jetable (27), pouvant s'engager de façon amovible dans ladite partie fixe, ladite partie amovible (27) englobant une conduite de fluide déformable (50), ledit appareil de réglage du débit englobant un moyen de restriction du débit (48, 52) pour restreindre le débit dans ladite conduite déformable (50), ladite partie amovible (27) pouvant être accouplée à un moyen de pompe (22) et à des conduites (23, 24) dans ledit système de manipulation du fluide, ledit appareil de réglage du débit du fluide comportant des voies de passage de fluide (60, 66, 68, 69, 82, 84) pour permettre l'écoulement du fluide entre des orifices d'admission et de sortie (20, 25) du dit appareil de réglage du débit du fluide, caractérisé en ce que ledit moyen de restriction du débit comprend un élément (52) sur la partie fixe (26) qui coopère avec un élément (48) sur la partie amovible (27) en vue de comprimer ladite conduite déformable, pour définir un pincement lorsque ladite partie amovible est engagée dans ladite partie fixe (27), en vue du réglage de la pression dans ledit système de manipulation du fluide (10).

2. Appareil de réglage d'un fluide selon la revendication 1, dans lequel ladite conduite déformable (50) est une conduite séparée flexible pouvant être accouplée entre deux orifices sur ladite partie amovible (27).

3. Appareil de réglage d'un fluide selon la revendication 1, dans lequel ladite conduite déformable est une conduite à moulage intégral, agencée entre deux orifices dans ladite partie amovible.

4. Appareil de réglage d'un fluide selon l'une quelconque des revendications précédentes, dans lequel la partie fixe (26) définit un évidement (32) en vue de la réception par glissement de ladite partie amovible (27).

5. Appareil de réglage d'un fluide selon l'une quelconque des revendications 1 à 4, dans lequel la partie amovible peut être engagée dans ladite partie fixe par une bride de serrage ou par un autre moyen de fixation approprié.

6. Appareil de réglage d'un fluide selon l'une quelconque des revendications précédentes, dans lequel la partie fixe (26) se compose de deux parties séparées, une première partie de guidage (28) et une deuxième partie de restriction du débit (30), la première partie de guidage (28) définissant un évidement (32) en vue de la réception par glissement de la partie amovible (27) et la deuxième partie de restriction du débit (30) comportant un élément (52) s'engageant dans un élément à réaction (48) sur ladite partie amovible (27), de sorte que la conduite déformable (50) est agencée entre les deux lors de l'engagement des parties fixe et amovible.

7. Appareil de réglage d'un fluide selon la revendication 6, dans lequel ledit appareil de réglage du débit du fluide englobe un système de surveillance du fluide, ledit système de surveillance du fluide comprenant une conduite (68), agencée dans ladite partie amovible (27), communiquant avec une conduite principale de fluide (66), ladite conduite (68) comportant un orifice de sortie (35a), ladite première partie de guidage (28) comportant un premier orifice d'admission (34a), aligné de façon étanche avec ledit orifice de sortie (35a), dans lequel est engagée ladite partie amovible (27), ledit premier orifice d'admission (34a) constituant l'entrée d'une conduite de prélèvement d'échantillons (69), ladite conduite de prélèvement d'échantillons (69) communiquant avec au moins une ouverture (86, 88) en vue de la réception d'un capteur (38, 40) destiné à mesurer un paramètre de fluide, ledit fluide échantillonné étant transféré à une sortie (74) dans ladite première partie de guidage (28).

8. Appareil de réglage d'un fluide selon la revendication 7, dans lequel plusieurs capteurs (38, 40) sont accouplés de façon amovible a ladite première partie de guidage (28) pour détecter différentes paramètres de fluide.

9. Appareil de réglage d'un fluide selon les revendications 7 ou 8, dans lequel ladite sortie (74) dans ladite première partie de guidage (28) est accouplée par l'intermédiaire d'un moyen de soupape (89) et d'une conduite (76) à une deuxième admission (78) dans ladite partie de guidage (28), ladite deuxième admission (78) dans ladite partie de guidage étant reconnectée par l'intermédiaire de conduites internes et de troisième et quatrième orifices (34b, 35b) à ladite partie amovible (27) et à un orifice de sortie de purge (84), l'agencement étant tel que le fluide de purge peut être transféré à travers ladite partie amovible (27) et ladite partie de guidage (28) lorsque ladite soupape (89) est ouverte.

10. Appareil de réglage d'un fluide selon l'une quelconque des revendications précédentes, dans lequel ledit appareil de réglage du débit du fluide est utilisé avec un système de séparation ou de filtrage.

11. Appareil de réglage d'un fluide selon la revendication 10, dans lequel le système de séparation est un système de dialyse servant à la désintoxication du sang.

12. Appareil de réglage d'un fluide selon la revendication 10, dans lequel ledit appareil de réglage du débit du fluide est utilisé avec un système de survie.

13. Appareil de réglage d'un fluide selon l'une quelconque des revendications 9 à 12, dans lequel ledit orifice de sortie (35a), ledit premier orifice d'admission (34a) et lesdits troisième et quatrième orifices d'admission et de sortie (34b, 35b) sont des orifices aseptiques.

14. Appareil de réglage d'un fluide selon l'une quelconque des revendications 7 à 13, dans lequel ladite partie amovible (27) est composée d'acier inoxydable de qualité médicale.

15. Appareil de réglage d'un fluide selon l'une quelconque des revendications 7 à 13, dans lequel ladite partie amovible (27) est composée de plastique stérilisable.

16. Appareil de réglage d'un fluide selon l'une quelconque des revendications précédentes, dans lequel la partie amovible (27) et une unité de filtre (16) sont combinées dans une seule structure intégrale (90), accouplée de façon amovible à la partie fixe.

17. Un système de séparation ou de filtrage de fluide, destiné à recevoir un fluide comportant au moins une substance devant en être séparée, ledit système comprenant un appareil de réglage du débit du fluide selon l'une quelconque des revendications précédentes, un moyen de pompe (22) pour pomper ledit fluide autour du dit système, ainsi qu'un moyen de séparation ou de filtrage (16) pour séparer ladite substance du dit fluide.

18. Un système selon la revendication 17, dans lequel ledit moyen de restriction du débit (48, 52), associé au dit appareil de réglage du débit (14), sert à régler la contre-pression dans ledit moyen de séparation.

19. Un système selon les revendications 17 ou 18, englobant un moyen de surveillance du fluide (38, 40) accouplé au dit appareil de réglage du débit (14) en vue de la surveillance d'au moins un paramètre de fluide.

20. Un système selon l'une quelconque des revendications 17 à 19, englobant un système de purge pour purger le fluide du dit moyen de surveillance du fluide (38, 40), ledit système de purge étant accouplé au dit appareil de réglage du débit du fluide (14), englobant un moyen de soupape (89).

21. Un système selon l'une quelconque des revendications 17 à 20, dans lequel le système de séparation est un système de dialyse, et dans lequel la partie stérilisable amovible (27) de l'appareil de réglage du débit est accouplée par glissement à un moyen de guidage (28) de ladite partie fixe (26) par l'intermédiaire d'orifices aseptiques (34a, 35a, 34b, 35b), un échantillon de sang pouvant ainsi être transféré au dit moyen de surveillance (38, 40) en vue d'une analyse.

22. Appareil de réglage du débit d'un fluide selon la revendication 1, destiné à être utilisé dans un système de dialyse, servant à la réception du sang à traiter, ladite parte stérilisable amovible (27) étant accouplée à un moyen de pompe (22) et à un élément de séparation ou de filtrage (16), ledit moyen de restriction du débit (48, 52) étant agencé de sorte à régler un niveau de contre-pression nécessaire à la dialyse, un moyen de surveillance du sang (38, 40) étant accouplé à ladite partie stérilisable amovible (27) et servant à recevoir un échantillon de sang transféré au dit élément de séparation ou de filtrage (16) pour analyser au moins un paramètre du dit sang, et un moyen de purge accouplé au dit appareil de réglage du débit étant destiné à être accouplé a une alimentation en fluide de purge après ladite dialyse, pour purger ledit moyen de surveillance du sang (38, 40), un moyen de réglage accouplé au dit appareil de réglage, au dit moyen de pompe (22) et au dit moyen de purge servant au contrôle de l'opération de dialyse et au réglage de la vitesse d'écoulement du sang et de la pression de séparation.

23. Appareil de réglage du débit d'un fluide selon la revendication 1, englobant un moyen de réglage de la pression, servant à régler la pression transmembranaire dans un dispositif de filtrage, le moyen de réglage de la pression comprenant un moyen de filtre (160) comportant une admission (158), destinée à recevoir un fluide d'admission à filtrer, et une sortie (174), destinée à recevoir le concentré du dit moyen de filtre (160), un premier moyen de surveillance de la pression (165), associé à une conduite d'admission pour mesurer la pression d'admission au dit moyen de filtre, un deuxième moyen de surveillance de la pression (184), associé avec une conduite de sortie, pour mesurer la pression du fluide de sortie, un moyen servant à comparer les pressions d'admission et de sortie pour fournir un signal de comparison, et un moyen de réglage du débit (48, 52) réagissant au signal de comparaison pour régler le débit du fluide à travers l'unité de filtre (160), pour optimaliser le réglage de la pression transmembranaire et du filtrage.

24. Appareil de réglage du débit d'un fluide selon la revendication 23, dans lequel le moyen de réglage du débit du fluide englobe un interrupteur de pression (167) agencé dans la conduite d'admission.

25. Appareil de réglage du débit d'un fluide selon la revendication 23, dans lequel le moyen de réglage du débit du fluide englobe un interrupteur de pression agencé dans la conduite de sortie.

26. Appareil de réglage du débit d'un fluide selon l'une quelconque des revendications 23 à 25, dans lequel les moyens de surveillance de la pression (165, 184) sont agencés dans des conduites de purge respectives (162b, 178b) des conduites d'admission et de sortie, de sorte que le fluide s'écoule le long des moyens de surveillance.

27. Appareil de réglage du débit d'un fluide selon l'une quelconque des revendications 23 à 25, dans lequel les moyens de surveillance de la pression (165, 184) soit des capteurs de pression à diaphragme, aucun contact n'existant ainsi entre le fluide en cours de traitement et l'appareil de surveillance de la pression.

**28.** Appareil de réglage du débit d'un fluide selon l'une quelconque des revendications 23 à 27, dans lequel les premier et deuxième moyens de surveillance de la pression (165, 184) sont respectivement accouplés à des première et deuxième soupapes électromagnétiques (166, 180), dont les sorties sont combinées dans une seule conduite (170a), les soupapes (166, 180) pouvant être actionnées pour purger le fluide d'un circuit de fluide de mesure associé.

**29.** Appareil de réglage du débit d'un fluide selon l'une quelconque des revendications 23 à 28, comportant une première pompe au niveau de ladite admission du filtre, une deuxième pompe agencée au niveau de la sortie du filtre et une troisième pompe agencée au niveau de la sortie du filtre, la sortie du produit de filtrage pouvant être accouplée à un réservoir d'une substance anticoagulante et ladite sortie du produit de filtrage étant connectée à une unité de collecte de plasma, l'appareil étant agencé de sorte à séparer le plasma du sang donné par un patient, de sorte qu'en service une première valeur de pression transmembranaire est utilisée pour actionner les première et troisième pompes, en vue de séparer le plasma et de stocker le plasma séparé dans une unité collectrice, et une deuxième valeur de pression transmembranaire étant utilisée pour arrêter les première et troisième pompes et pour actionner la deuxième pompe, en vue de restituer le sang au patient.

FIG.1

FIG.2

FIG. 3

FIG. 4

FIG. 5

FIG. 6

17

FIG. 7

FIG. 7a

FIG. 8

FIG. 9